(19) Europäisches Patentamt
European Patent Office
Office européen des brevets

(11) Veröffentlichungsnummer : **0 047 973**
**B1**

(12) # EUROPÄISCHE PATENTSCHRIFT

(45) Veröffentlichungstag der Patentschrift : **05.06.85**

(51) Int. Cl.⁴ : **C 07 C143/80**, C 07 D307/52, C 07 C139/00, C 07 C147/12

(21) Anmeldenummer : **81107071.3**

(22) Anmeldetag : **09.09.81**

(54) **Verfahren zur Herstellung von 5-Sulfamoyl-orthanilsäuren.**

(30) Priorität : **16.09.80 DE 3034880**

(43) Veröffentlichungstag der Anmeldung : **24.03.82 Patentblatt 82/12**

(45) Bekanntmachung des Hinweises auf die Patenterteilung : **05.06.85 Patentblatt 85/23**

(84) Benannte Vertragsstaaten : **AT CH DE LI NL SE**

(56) Entgegenhaltungen : **DE-A- 2 718 871**

(73) Patentinhaber : **HOECHST AKTIENGESELLSCHAFT**
**Postfach 80 03 20**
**D-6230 Frankfurt am Main 80 (DE)**

(72) Erfinder : **Sturm, Karl, Dr.**
**Berndesallee 64**
**D-6501 Heidesheim (DE)**

**EP 0 047 973 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein Verfahren zur Herstellung von 5-Sulfamoyl-orthanilsäuren der allgemeinen Formel I in Form ihrer Alkali- oder Aminsalze.

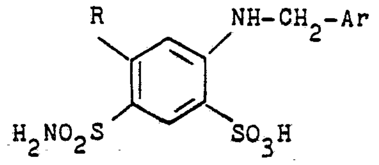

(I)

In der Formel bedeuten :

R N-Methylanilino, Phenylsulfonyl, ggf. durch Cl, $CH_3$ oder $OCH_3$ substituiertes Phenoxy oder Alkylsulfonyl, Cycloalkylsulfonyl oder Cycloalkylalkylsulfonyl mit je bis zu 10 Kohlenstoffatomen ;

Ar Phenyl oder Furyl

Als Salze dieser Säuren kommen vorzugsweise die Natrium-, Kalium- oder Ammoniumsalze, daneben aber auch die Salze mit primären, sekundären und tertiären Aminen, und auch mit basischen Arzneimitteln in Frage.

Die Verfahrensprodukte sind teils als hochwirksame Salidiuretika bekannt (DBP 27 18 871) teils in den Patentanmeldungen P 30.06.686.5 (HOE 80/F 029) und P 30.09.229.6 (HOE 80/F 049) beschrieben.

Das Verfahren ist dadurch gekennzeichnet, daß man einen Arylester der allgemeinen Formel II

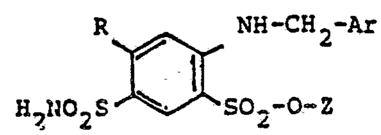

(II)

worin Z Aryl, vorzugsweise Phenyl, Tolyl oder Xylyl bedeutet, in einem hydrierbeständigen organischen Lösungsmittel und unter Zusatz von mindestens einem Neutralisationsäquivalent eines Alkali hydroxids, Ammoniak oder eines hydrierbeständigen primären, sekundären oder tertiären Amins in Gegenwart eines Palladiumkatalysators bis zur Aufnahme von einem Moläquivalent Wasserstoff hydriert.

Die Hydrierung verläuft bereits bei Raumtemperatur sehr rasch und führt zur Spaltung in die entsprechende Sulfosäure, die durch die zugesetzte Base neutralisiert wird, und den entsprechenden Kohlenwasserstoff Z-H, das heißt in der Regel Benzol, Toluol oder einem der isomeren Xylole. Generell läßt sich nach dem erfindungsgemäßen Verfahren jeder aromatische oder heteroaromatische Rest Z abspalten, es bringt jedoch technisch keinen Vorteil andere als die obengenannten Phenyl- und methylsubstituierten Phenylester als Ausgangsmaterial einzusetzen. Außerdem ist zu berücksichtigen, daß verschiedene Arylreste Z, insbesondere heteroaromatische Systeme, unter den angewandten Reaktionsbedingungen nach der Esterspaltung weiter hydriert werden können und daß dadurch der Endpunkt der Spaltung nur schwer zu erkennen ist. So wird beispielsweise Z = Naphthyl zu Tetrahydronaphtalin, Z = Pyridyl zu Piperidin und Z = Chinolyl zu Tetrahydrochinolin hydriert.

Als Lösungsmittel verwendet man bei dem erfindungsgemäßen Verfahren mit besonderem Vorteil mit Wasser mischbare Alkohole, Äther, Hydroxyäther oder Amide, beispielsweise Methanol, Äthanol, Isopropanol, Tetrahydrofuran, Dioxan, Glykolmonomethyläther, Diäthylenglykoldimethyläther, Dimethylformamid, Dimethylacetamid, sowie Mischungen dieser Lösungsmittel miteinander, gegebenenfalls unter Zusatz von Wasser.

Als Palladiumkatalysator kommen vorzugsweise Palladiummohr oder Palladium 1- bis 10-prozentig auf einem Träger wie Aktivkohle, Aluminiumoxid und Bariumsulfat in Frage.

Um den Vorteil des Verfahrens zu nutzen, die Endprodukte direkt frei von Fremdsalzen gewinnen zu können, setzt man nicht flüchtige Basen wie anorganische Basen oder höhermolekulare Amine vorteilhaft nur in der zur Neutralisation der gebildeten Sulfosäure berechneten Menge zu. Flüchtige Basen wie Ammoniak und oder niedrigsiedende Amine können auch ohne Nachteile im Überschuß verwendet werden.

Besonders vorteilhaft ist die hydrierende Spaltung in Gegenwart von überschüssigem Ammoniak, einmal weil der Überschuß Ammoniak schon beim Eindampfen der Reaktionslösung vollständig entfernt wird, zum zweiten, weil das isolierte Ammoniumsalz durch Behandeln mit der berechneten Menge einer anderen Base leicht in jedes gewünschte Amin- oder Metallsalz übergeführt werden kann.

Die hydrogenolytische Esterspaltung verläuft bereits bei Raumtemperatur und einem geringen Wasserstoffüberdruck von 0,1 bis 0,2 bar sehr rasch und mit scharf erkennbarem Endpunkt. Hydrierungen in kleinerem Maßstab, bis etwa 2 Mol, werden daher vorteilhaft in der Schüttelente ausgeführt. Bei größeren Ansätzen ist es vorteilhaft im Autoklaven, bei Wasserstoffdrücken zwischen 0,2 und etwa 2 bar zu arbeiten.

Nach beendeter Esterspaltung erfolgt sehr viel langsamer die hydrogenolytische Abspaltung der

Arylmethylgruppe vom Stickstoff in der 2-Position unter Bildung von Ar—$CH_3$ und der entsprechenden primären Aminoverbindung. Der Unterschied in der Hydriergeschwindigkeit ist jedoch so groß, daß der Endpunkt der Esterhydrierung exakt zu erkennen ist.

Nach Abtrennen des Katalysators und Eindampfen der farblosen Hydrierlösung liegt das Endprodukt bereits in sehr reiner Form vor. Alkalisalze, die mit der äquivalenten Menge Base erhalten wurden, verreibt man vorteilhaft mit absolutem Äthanol oder Isopropanol bei Raumtemperatur und saugt ab oder kristallisiert sie aus wäßrigem Alkohol oder auch aus Wasser um. Die wesentlich leichter löslichen Amin- und Ammoniumsalze werden vorteilhaft aus Essigester durch portionsweise Zugabe von Diisopropyläther oder Petroläther kristallin gefällt. Die Ausbeuten liegen in der Regel über 90 % der Theorie.

Die hydrogenolytische Spaltung von Sulfosäurearylestern mittels Palladium ist mehrfach in der Literatur beschrieben. Der glatte Verlauf der erfindungsgemäßen Hydrierung war jedoch daraus nicht abzuleiten, da auch die hydrogenolytische Abspaltung einer Arylmethylgruppe vom Stickstoff eine dem Fachmann geläufige Reaktion ist und nicht vorauszusehen war, welche der beiden möglichen Spaltreaktionen in dem Molekül II bevorzugt sein würde.

Insbesondere ist aus Chem. Ber. *99*, 345-352 (1966) bekannt, daß die den Ausgangsstoffen der allgemeinen Formel II nahe verwandten Verbindungen IIIa bis IIIc

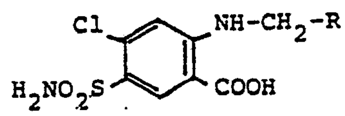

IIIa R = 2-Furyl
IIIb R = 2-Thienyl
IIIc R = Phenyl

durch Hydrierung mit Palladium in Methanol leicht in 4-Chlor-5-sulfamoylanthranilsäure und R—$CH_3$ gespalten werden bzw. IIIa bei der Palladiumhydrierung in Tetrahydrofuran, Dioxan oder Essigester zum entsprechenden Tetrahydrofurfurylderivat hydriert wird. Diese Konkurrenzreaktionen spielen bei dem erfindungsgemäßen Verfahren überraschenderweise keine Rolle.

### Beispiel 1

N-(2-Furylmethyl)-4-phenoxy-5-sulfamoyl-orthanilsäure-natrium

Die Lösung von 10,0 g N-(2-Furylmethyl)-4-phenoxy-5-sulfamoyl-orthanilsäurephenylester (20 mMol) in 100 ml Tetrahydrofuran und 50 ml Methanol wird mit 20 ml 1n NaOH und nach Zugabe von frischem Palladiummohr bei Raumtemperatur in der Schüttelente bei 0,1 bar Wasserstoffdruck hydriert. Nach 10 Minuten und einem Verbrauch von 520 ml Wasserstoff kommt die Hydrierung zum Stehen. Nach Abtrennen des Katalysators dampft man das farblose Filtrat zur Trockne ein, verreibt den festen Rückstand mit 100 ml Isopropanol, saugt nach kurzem Stehen bei Raumtemperatur ab und trocknet das Endprodukt bei 100 °C.

8,5 g farblose Kristalle (95 % d. Th.), Zers.-P. 232 °C

Die gleiche Ausbeute erhält man, wenn man bei obigem Ansatz an Stelle des Palladiumohrs 10-proz. Pd auf Kohle verwendet. Die Aufnahme ist nach etwa 5 Min. und einem Verbrauch von 530 ml Wasserstoff beendet. An dieser Stelle bricht man die Hydrierung ab und arbeitet wie oben beschrieben auf.

### Beispiel 2

N-(2-Furylmethyl)-4-phenoxy-5-sulfamoyl-orthanilsäureammonium

Die Hydrierung erfolgt analog Beispiel 1 mit 20 ml 8n wässrigem Ammoniak an Stelle der Natronlauge und 10-prozentigem Palladium auf Kohle als Katalysator. Nach 5 Minuten und einem Verbrauch von 500 ml Wasserstoff wird die Aufnahme plötzlich sehr langsam. Man bricht die Hydrierung ab, saugt vom Katalysator ab und dampft das Filtrat zur Trockne ein. Beim Aufkochen des festen Rückstands mit 100 ml Essigester entsteht kurz eine klare Lösung, aus der nach wenigen Minuten bereits in der Wärme das Endprodukt rasch auskristallisiert. Nach einstündigem Stehen bei Raumtemperatur saugt man ab und trocknet auf dem Dampfbad.

8,1 g farblose Prismen (93 % d. Th.), Zers.-P. 190 °C.

### Beispiel 3

N-(2-Furylmethyl)-4-phenoxy-5-sulfamoyl-orthanilsäurekalium

10,3 g 2-(2-Furylmethyl)-4-phenoxy-5-sulfamoylorthanilsäure-4-tolylester (20 mMol) werden in einer Mischung von 10 ml Dimethylformamid und 90 ml Methanol in Gegenwart von 5-prozentigem Palladium auf Bariumsulfat und unter Zusatz von 20 ml 1n KOH in der Schüttelente bei Raumtemperatur hydriert. Nach 20 Minuten und einer Aufnahme von 530 ml Wasserstoff wird die Hydrierung plötzlich sehr langsam.

Man bricht ab, trennt vom Katalysator ab, und dampft die farblose Hydrierlösung zur Trockne ein. Nach verreiben des Rückstands mit 80 ml Essigester, saugt man ab und trocknet bei 100 °C.

8,8 g farblose Kristalle (95 % d. Th.), Zers.-P. 222 °C.

Beispiel 4

N-(2-Furylmethyl)-4-(N-methylanilino)-5-sulfamoylorthanilsäureammonium

10,3 g N-(2-Furylmethyl)-4-(N-methylanilino)-5-sulfamoylorthanilsäurephenylester (20 mMol) werden in einer Mischung von 150 ml Tetrahydrofuran, 50 ml Methanol und 20 ml 8n wäßrigem Ammoniak mit 10-prozentiger Palladiumkohle bei Raumtemperatur in der Schüttelente hydriert. Nach Aufnahme von 500 ml Wasserstoff in etwa 30 Minuten wird die Hydrierung plötzlich sehr langsam. Man bricht ab, filtriert und dampft die Hydrierlösung ein. Das Endprodukt wird in 50 ml Essigester gelöst und durch portionsweise Zugabe von 100 ml Diisopropyläther kristallin gefällt.

7,8 g farblose Plättchen (91 % d. Th.), Zers.-P. 185 °C.

Beispiel 5

N-(2-Furylmethyl)-4-isobutylsulfonyl-5-sulfamoylorthanilsäure-natrium

10,6 g N-(2-Furylmethyl)-4-isobutylsulfonyl-5-sulfamoylorthanilsäurephenylester (20 mMol) werden analog Beispiel 4 mit 20 ml 1n NaOH an Stelle des Ammoniaks hydriert. Nach Eindampfen der Hydrierlösung wird der Rückstand aus wenig Wasser umkristallisiert.

8,8 g farblose Prismen (92 % d. Th.), Zers.-P. oberhalb 300 °C.

Beispiel 6

N-Benzyl-4-phenoxy-5-sulfamoylorthanilsäure-ammonium

10,2 g N-Benzyl-4-phenoxy-5-sulfamoylorthanilsäure-phenylester (20 mMol) werden in Gegenwart von 10-prozentiger Palladiumkohle in einer Mischung von 100 ml Tetrahydrofuran und 30 ml 10n wäßrigem Ammoniak bei Raumtemperatur in der Schüttelente hydriert. Nach Aufnahme von 550 ml Wasserstoff in etwa einer Stunde wird die Hydrierung plötzlich sehr langsam. Man trennt jetzt vom Katalysator ab, dampft die Hydrierlösung ein und kristallisiert den Rückstand aus 100 ml Essigester um.

7,6 g schuppige Kristalle (83 % d. Th.), Zers.-P. 220 °C.

**Patentansprüche**

1. Verfahren zur Herstellung von 5-Sulfamoyl-orthanilsäuren der Formel I

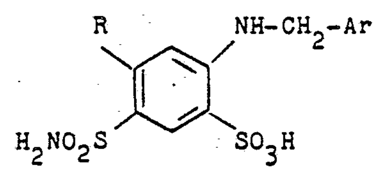

(I)

in der bedeuten

R N-Methylanilino, Phenylsulfonyl, ggf. durch Cl, $CH_3$ oder $OCH_3$ substituiertes Phenoxy oder Alkylsulfonyl, Cycloalkylsulfonyl oder Cycloalkylalkylsulfonyl mit je bis zu 10 Kohlenstoffatomen

Ar Phenyl oder Furyl

in Form ihrer Salze dadurch gekennzeichnet, daß man einen Arylester der Formel II

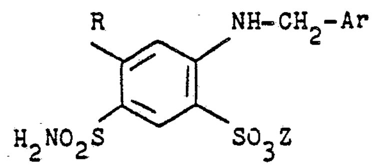

(II)

in der Z Aryl bedeutet, in einem hydrierbeständigen Lösungsmittel unter Zusatz von mindestens einem Äquivalent einer Base in Gegenwart eines Palladiumkatalysators bis zur Aufnahme eines Moläquivalents Wasserstoff hydriert.

2. Verfahren nach Anspruch 1, dadurch gekennzeichnet, daß von einem Arylester der Formel II ausgegangen wird, in dem Z für Phenyl, Tolyl oder Kresyl steht.

3. Verfahren nach Anspruch 1, dadurch gekennzeichnet, daß als Base ein Alkalihydroxid, Ammoniak oder ein hydrierbeständiges Amin verwendet wird.

**Claims**

1. A process for the preparation of 5-sulfamoyl-orthanilic acids of the formula I

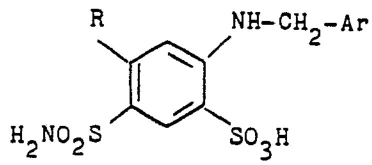

(I)

in which

R denotes N-methylanilino, phenylsulfonyl, phenoxy, which is optionally substituted by Cl, $CH_3$ or $OCH_3$, or alkyl sulfonyl or cycloalkylsulfonyl or cycloalkylalkylsulfonyl having up to 10 carbon atoms in each case, and

Ar denotes phenyl or furyl, in the form of their salts, which comprises hydrogenating an aryl ester of the formula II

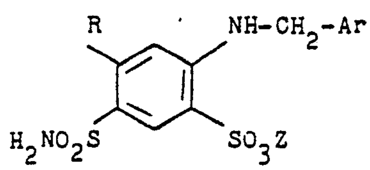

(II)

in which Z denotes aryl, in a solvent which is stable to hydrogenation and with the addition of at least one equivalent of a base, in the presence of a palladium catalyst, until one mol equivalent of hydrogen has been absorbed.

2. A process as claimed in Claim 1, wherein the starting material is an aryl ester of the formula II, in which Z represents phenyl, tolyl or cresyl.

3. A process as claimed in Claim 1, wherein an alkali metal hydroxide, ammonia or an amine which is stable to hydrogenation is used as the base.

**Revendications**

1. Procédé de préparation d'acides 5-sulfamoylorthaniliques de formule I

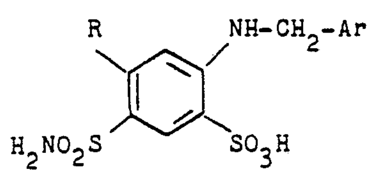

(I)

dans laquelle

R désigne un groupe N-méthylanilino, phénylsulfonyle, phénoxy éventuellement substitué par Cl, $CH_3$ ou $OCH_3$ ou un groupe alkylsulfonyle, cycloalkylsulfonyle ou cycloalkylalkylsulfonyle ayant à chaque fois jusqu'à 10 atomes de carbone

Ar représente un groupe phényle ou furyle sous forme de leurs sels caractérisé en ce qu'on effectue une hydrogénation d'un ester arylique de formule II

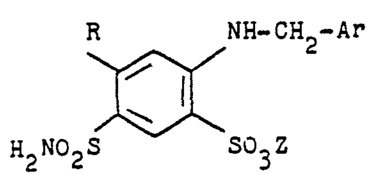

(II)

dans laquelle Z désigne un groupe aryle, dans un solvant résistant à l'hydrogénation avec addition d'au moins un équivalent d'une base, en présence d'un catalyseur à base de palladium, jusqu'à la fixation d'un équivalent molaire d'hydrogène.

2. Procédé selon la revendication 1, caractérisé en ce qu'on part d'un ester arylique de formule II dans laquelle Z désigne un groupe phényle, tolyle, ou crésyle.

3. Procédé selon la revendication 1, caractérisé en ce que l'on utilise comme base un hydroxyde alcalin, de l'ammoniaque ou une amine résistant à l'hydrogénation.